# EUROPEAN PATENT APPLICATION

(11) **EP 2 754 401 A2**
(43) Date of publication of application: **16.07.2014**
(21) Application number: 12829241.4
(22) Date of filing: 05.09.2012
(51) Int. Cl.: A61B 17/29, A61B 17/00

(54) **MINIMALLY INVASIVE SURGICAL INSTRUMENT HAVING DETACHABLE END EFFECTOR**

(30) Priority: 08.09.2011 KR 20110091515
(71) Applicant: Movasu, Inc., Seoul 110-450 (KR)
(72) Inventor: JEONG, Chang Wook, Seoul 110-450 (KR); SIN, Chun Chol, Seoul 110-450 (KR); KIM, Sung Ryul, Seoul 110-450 (KR); KIM, Hyung Tae, Seoul 110-450 (KR)
(74) Representative: Zardi, Marco
(86) International application number: PCT/KR2012/007094
(87) International publication number: WO 2013/036024

(57) **Abstract**

The present invention relates to a minimally invasive surgical instrument having a detachable end effector. Provided is a minimally invasive surgical instrument comprising a shaft, a joint section which is connected to one end of the shaft, and an end effector which is connected to the joint section and thus is capable of articulatory movement. Therein, the joint section comprises a detachable connection section which can receive rotational torque, and the end effector comprises an operational end-section connection unit which can be attached to the detachable connection section by being screwed in, or can be separated from the detachable connection section by being unscrewed.

## Description

### FIELD OF THE INVENTION

The present invention relates to a minimally invasive surgical instrument having a detachable end effector.

### BACKGROUND

Minimally invasive surgery is a surgical approach that involves the use of instruments inserted through several tiny incision openings to perform a surgery causing minimal tissue trauma in human or animal bodies.

The minimally invasive surgery relatively reduces changes in metabolism of a patient in the period of post-surgical care, so it facilitates rapid recovery of the patient. Therefore, the minimally invasive surgery shortens the length of hospitalization of the patient after the surgery and allows the patient to return to normal physical activities in a short period of time. In addition, the minimally invasive surgery causes less pain and leaves fewer scars on the patient's body after the surgery.

One of the general forms of the minimally invasive surgery is endoscopy. Among the others, a laparoscopy that involves minimally invasive inspection and operation inside abdominal cavity is known as the most general form of endoscopy. To operate a standard laparoscopic surgery, the abdomen of the patient is insufflated with gas and at least one small incision is formed to provide an entrance for laparoscopic surgical instruments, through which a trocar is inserted. When performing the surgery, it is general that a user puts the laparoscopic surgical instruments into a surgical site or the like through the trocar, and manipulates (or controls) the instruments from the outside of abdominal cavity. In general, the laparoscopic surgical instruments include a laparoscope (for observation of a surgical site) and other working tools. Herein, the working tools are similar to the conventional tools used for small incision surgery, except that the end effector or working end of each tool is separated from its handle or the like by a shaft. For instance, the working tools may include a clamp, a grasper, scissors, a stapler, a needle holder, and so forth. Meanwhile, the user monitors the procedure of the surgery through a monitor that displays the images of the surgical site which are taken by the laparoscope. The endoscopic approaches similar to the above are broadly used in retroperitoneoscopy, pelviscopy, arthroscopy, cisternoscopy, sinuscopy, hysteroscopy, nephroscopy, cystoscopy, urethroscopy, pyeloscopy, and so on.

The inventor(s) has developed various minimally invasive surgical instruments useful for the above-mentioned minimally invasive surgeries and has already disclosed the features of the structures and effects of the same in Korean Patent Application Nos. 2008-51248, 2008-61894, 2008-79126 and 2008-90560, the contents of which are incorporated herein by reference in its entirety. Additionally, the inventor(s) have also introduced a minimally invasive surgical instrument with improved functionality, which is more advantageous for users and patients, in Korean Patent Application Nos. 2010-115152, 2011-3192, 2011-26243 and 2011-29771, the contents of which are incorporated herein by reference in its entirety.

Herein, the inventor(s) now present a minimally invasive surgical instrument to and from which a user may easily attach and detach an end effector.

### SUMMARY OF THE INVENTION

One object of the present invention is to provide a minimally invasive surgical instrument to and from which a user may easily attach and detach an end effector.

Another object of the invention is to provide a minimally invasive surgical instrument which may be more economically used by increasing the utility and reusability of the parts except the end effector thereof.

According to one aspect of the invention to achieve the objects as described above, there is provided a minimally invasive surgical instrument comprising a shaft; a joint unit being connected to one end of the shaft; and an end effector being connected to the joint unit and capable of carrying out joint motion thereby, wherein the joint unit comprises a detachable connecting unit being capable of receiving rotational torque, and wherein the end effector comprises a working end connecting unit being capable of being screwed into the detachable connecting unit to attach thereto or being screwed out of the detachable connecting unit to detach therefrom.

In addition, there may be provided other configurations to implement this invention.

According to the invention, there is provided a minimally invasive surgical instrument to and from which a user may easily attach and detach an end effector.

According to the invention, there is provided a minimally invasive surgical instrument which may be more economically used by increasing the utility and reusability of the parts except the end effector thereof.

### BRIEF DESCRIPTION OF THE DRAWINGS

Fig. 1 shows the overall appearance of a minimally invasive surgical instrument according to one embodiment of the invention.
Fig. 2 is a perspective view of some components such as an end effector 200 and a joint unit 300 of a minimally invasive surgical instrument according to one embodiment of the invention.
Fig. 3 is an exploded perspective view of the components of the minimally invasive surgical instrument shown in Fig. 2.

### DETAILED DESCRIPTION OF THE PREFERRED EMBODIMENTS

In the following detailed description of the invention, references are made to the accompanying drawings that show, by way of illustration, specific embodiments in which the invention may be practiced. These embodiments are described in sufficient detail to enable those skilled in the art to practice the invention. It is to be understood that the various embodiments of the invention, although different from each other, are not necessarily mutually exclusive. For example, specific shapes, structures, or characteristics described herein may be implemented as modified from one embodiment to another embodiment without departing from the spirit and the scope of the invention. Furthermore, it shall be understood that the locations or arrangements of individual elements within each embodiment may be also modified without departing from the spirit and the scope of the invention. Therefore, the following detailed description is not to be taken in a limiting sense, and the scope of the invention is to be taken as encompassing the scope of the appended claims and all equivalents thereof. In the drawings, like reference numerals refer to the same or similar elements throughout the several views.

Hereinafter, preferred embodiments of the present invention will be described in detail with reference to the accompanying drawings to enable those skilled in the art to easily implement the invention.

Meanwhile, it should be understood that the term "connection" herein encompasses a direct connection or an indirect connection (i.e., via separate components) between mechanical or other types of components.

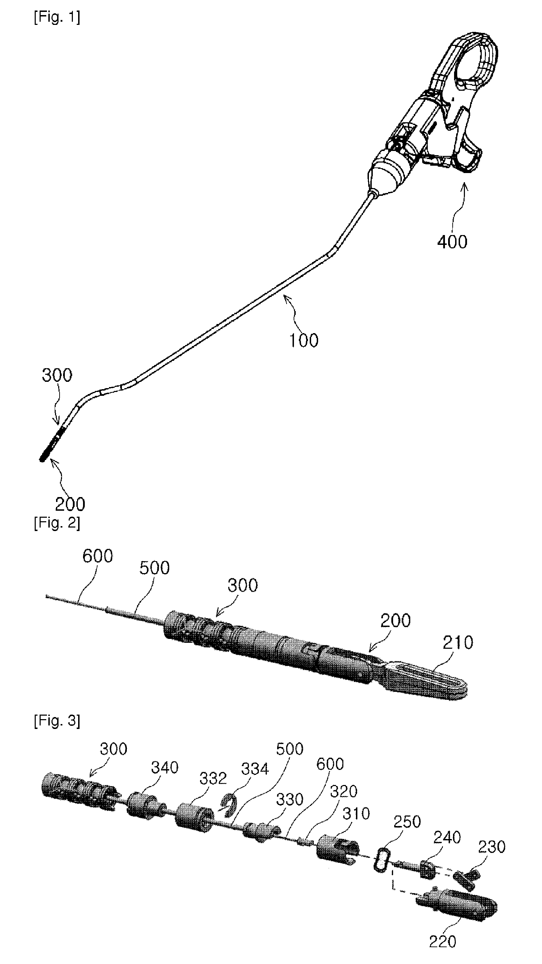

Fig. 1 shows the overall appearance of a minimally invasive surgical instrument according to one embodiment of the invention.

Reference will be made to Fig. 1. The minimally invasive surgical instrument may comprise a shaft 100; an end effector 200 being connected to one end of the shaft 100 to perform surgery by using surgical tools (not shown) or functioning itself as a surgical tool; a joint unit 300 to connect the shaft 100 and the end effector 200 and to provide the end effector 200 with joint functionality; and a handling unit 400 being connected to the other end of the shaft 100 and capable of being held and manipulated by a user.

First, the shaft 100 may include a cavity therein to support and pass at least one wire or torque transmission member, in the same manner as those of the minimally invasive surgical instruments disclosed in the aforementioned Korean patent applications. (The torque transmission member is mainly intended for the rolling motion of the end effector 200, while the shaft 100 may function itself as the torque transmission member in some cases.) The shaft 100 may comprise at least one segment as necessary. Further, the shaft 100 may comprise a bend in at least a part thereof.

Next, the end effector 200 may carry out joint motion, rolling motion, opening/closing motion and the like by the action of the at least one wire or torque transmission member passing from the handling unit 400 to the joint unit 300 via the shaft 100, in the same manner as those of the minimally invasive surgical instruments disclosed in the aforementioned Korean patent applications. The tip of the end effector 200 may be implemented in the form of a clamp, a grasper, a pair of scissors, a stapler, a needle holder, a hook-type electrode or the like.

Next, the joint unit 300 may act together with the at least one wire or torque transmission member to allow the end effector 200 to carry out joint motion, rolling motion and the like, in the same manner as those of the minimally invasive surgical instruments disclosed in the aforementioned Korean patent applications.

Finally, the handling unit 400 may control the joint motion, rolling motion, opening/closing motion and the like of the end effector 200 according to the user's manipulation, in the same manner as those of the minimally invasive surgical instruments disclosed in the aforementioned Korean patent applications. To allow for such control, the at least one wire or torque transmission member may be connected to the handling unit 400.

Fig. 2 is a perspective view of some components such as the end effector 200 and the joint unit 300 of the minimally invasive surgical instrument according to one embodiment of the invention. Further, Fig. 3 is an exploded perspective view of the components of the minimally invasive surgical instrument shown in Fig. 2.

Reference will be made to Fig. 2. As described above, the end effector 200 and the joint unit 300 may be connected to each other, and a torque transmission member 500 or an opening/closing wire 600 may be supported and passed through some part of the joint unit 300 (preferably the longitudinal central axis thereof) toward the end effector 200. In connection with the configuration of the joint unit 300, the torque transmission member 500 and the opening/closing wire 600, further reference may be made to Korean Patent Application Nos. 2011-86738 and 2011-89854, the contents of which are incorporated herein by reference in its entirety.

Reference will be made to Fig. 3.

First, the end effector 200 may essentially comprise a working end 210 (shown in Fig. 2), a working end connecting unit 220 and an X-shaped link 230. The configuration and operating principle of the end effector 200 may be similar to those of the end effectors disclosed in the aforementioned Korean patent applications.

Further, the end effector 200 may further comprise an opening/closing wire fixing unit 240 and a fixing ring 250.

Since the opening/closing wire fixing unit 240 may be connected with the X-shaped link 230 and also with an opening/closing wire cap 320 to be described below, it may pull the X-shaped link 230 to close the working end 210 as the opening/closing wire 600 is pulled. In connection with this, it should be noted that those skilled in the art may also employ an alternative configuration in which the working end 210 is opened as the opening/closing wire 600 is pulled, or in which the working end 210 is closed or opened by means of a component that may apply a pushing force in place of the opening/closing wire 600.

The fixing ring 250 may be made of an elastic material or other materials, and used to wind the opening/closing wire fixing unit 240 and the opening/closing wire cap 320 together to connect and fix them to each other.

The components of the end effector 200 will be further discussed when those of the joint unit 300 are described below.

The configuration and operating principle of the joint unit 300 may be essentially similar to those of the joint units disclosed in Korean Patent Application Nos. 2011-86738 and 2011-89854.

In addition to the above configuration, the joint unit 300 may be configured to further comprise a detachable connecting unit 310, an opening/closing wire cap 320, a rolling connecting unit 330, a holder 332, a fastener 334, and a connecting unit 340.

The detachable connecting unit 310 may comprise a groove having a P-shape, r-shape or similar shape into which a protrusion of the working end connecting unit 220 may be screwed as necessary. Thus, at least a part of the working end connecting unit 220 may be fitted and fixed to the detachable connecting unit 310. (That is, the end effector 200 may be fixed to the detachable connecting unit 310.) In this case, the working end connecting unit 220 and the working end 210 attached thereto may operate together in a roll direction when the detachable connecting unit 310 operates in the roll direction. (That is, the roll direction operation of the end effector 200 may be caused.) To allow for such series of operations, the detachable connecting unit 310 may be fixed to the rolling connecting unit 330, which may receive the rotational torque of the torque transmission member 500 as will be described below. Meanwhile, the working end connecting unit 220 may be screwed in the opposite direction and detached from the detachable connecting unit 310, as necessary. (That is, the end effector 200 may be separated from the detachable connecting unit 310.)

The opening/closing wire cap 320 may be connected to the opening/closing wire fixing unit 240 while grasping the opening/closing wire 600. To allow for such connection, the opening/closing wire fixing unit 240 and the opening/closing wire cap 320 may comprise some groove-protrusion structures that may be disposed facing each other. In order to fix the above connection, the fixing ring 250 may be used in the connecting region between the opening/closing wire fixing unit 240 and the opening/closing wire cap 320.

The rolling connecting unit 330 may comprise one end having the shape as shown, which may engage with one end of the working end connecting unit 220, so that it may be connected to and fixed together with the working end connecting unit 220 in the detachable connecting unit 310. (This fixation may be achieved by screwing in the working end connecting unit 220 as it is engaged with the rolling connecting unit 330.) Further, the rolling connecting unit 330 may comprise the other end having such a shape that it may be fixed to the connecting unit 340 to be described below, with the holder 332 and the fastener 334 as shown being interposed therebetween. Further reference may be made to Korean Patent Application Nos. 2011-86738 and 2011-89854 in connection with the illustrative principle of connecting the rolling connecting unit 330 and the connecting unit 340. Meanwhile, the rolling connecting unit 330 may be configured such that the torque transmission member 500 is joined and fixed to the above end. Accordingly, the rolling connecting unit 330 may receive the rotational torque of the torque transmission member 500 to cause the roll direction operation of the end effector 200. Meanwhile, the rolling connecting unit 330 may further comprise a passage to at least allow the opening/closing wire 600 to be extended and passed to the opening/closing wire cap 320.

The connecting unit 340 may be configured to constitute a part of the joint unit 300 and achieve such joint motion as discussed in Korean Patent Application No. 2011-86738 or 2011-89854. The connecting unit 340 may comprise a passage to allow the torque transmission member 500 to operate freely in the roll direction.

### Applications

According to an application of the present invention, those skilled in the art may partially change the form and such of the handling unit or the like so that the wire or torque transmission member of the minimally invasive surgical instrument may be operated by an electric motor or the like of another motor-based system (not shown) such as a surgical robot, as necessary.

Although the present invention has been described in terms of specific items such as detailed elements as well as the limited embodiments and the drawings, they are only provided to help general understanding of the invention, and the present invention is not limited to the above embodiments. It will be appreciated by a person of ordinary skill in the art that various modifications and changes may be made from the above description.

Therefore, the spirit of the present invention shall not be limited to the above-described embodiments, and the entire scope of the appended claims and their equivalents will fall within the scope and spirit of the invention.

## Claims

1. A minimally invasive surgical instrument comprising:
a shaft;
a joint unit being connected to one end of the shaft; and
an end effector being connected to the joint unit and capable of carrying out joint motion thereby,
wherein the joint unit comprises a detachable connecting unit being capable of receiving rotational torque, and
wherein the end effector comprises a working end connecting unit being capable of being screwed into the detachable connecting unit to attach thereto or being screwed out of the detachable connecting unit to detach therefrom.

2. A minimally invasive surgical instrument as claimed in Claim 1, further comprising an opening/closing wire, wherein the joint unit further comprises an opening/closing wire cap being pulled together with the opening/closing wire when the wire is pulled while the cap is grasping the wire.

3. A minimally invasive surgical instrument as claimed in Claim 2, wherein the end effector further comprises an opening/closing wire fixing unit being connected to a working end thereof, and
wherein the opening/closing wire fixing unit is connected to the opening/closing wire cap.

4. A minimally invasive surgical instrument as claimed in Claim 3, wherein the connection between the opening/closing wire fixing unit and the opening/closing wire cap is achieved by means of a groove-protrusion structure or a fixing ring.

5. A minimally invasive surgical instrument as claimed in Claim 1, wherein the joint unit further comprises a rolling connecting unit being capable of transmitting the rotational torque to the detachable connecting unit.

6. A minimally invasive surgical instrument as claimed in Claim 5, wherein one end of the rolling connecting unit has a shape to engage with one end of the working end connecting unit.

7. A minimally invasive surgical instrument as claimed in Claim 6, wherein the working end connecting unit and the rolling connecting unit are capable of being screwed together into the detachable connecting unit as their respective ends are engaged with each other.

8. A minimally invasive surgical instrument as claimed in Claim 5, wherein one end of the rolling connecting unit is connected to a joint motion element of the joint unit with a holder and a fastener being interposed therebetween.
